# EUROPEAN PATENT SPECIFICATION

(11) **EP 2 933 244 B1**
(45) Date of publication and mention of the grant of the patent: **23.11.2016**
(21) Application number: 13863577.6
(22) Date of filing: 10.12.2013
(51) Int. Cl.: C07C 69/732

(54) **FLUORINE ATOM-CONTAINING PHENOL COMPOUND**
FLUORATOMHALTIGE PHENOLVERBINDUNG
COMPOSÉ PHÉNOLIQUE CONTENANT UN ATOME DE FLUOR

(30) Priority: 13.12.2012 JP 2012272329
(43) Date of publication of application: 21.10.2015
(73) Proprietor: Fujifilm Corporation, Minato-ku Tokyo 106-8620 (JP)
(72) Inventor: OYA, Toyohisa, Ashigara-kami-gun Kanagawa 258-8577 (JP); MATSUMURA, Tokihiko, Ashigara-kami-gun Kanagawa 258-8577 (JP); MATSUSHITA, Yasuaki, Ashigara-kami-gun Kanagawa 258-8577 (JP)
(74) Representative: Hoffmann Eitle
(86) International application number: PCT/JP2013/083126
(87) International publication number: WO 2014/092097

(56) References cited:
- JP-A- H0 641 018
- JP-A- H05 247 960
- JP-A- H08 325 465
- JP-A- H11 349 819
- LE STANG S ET AL: "Convenient syntheses of fluorous phenols of the formula HOC6H5-n((CH2)3(CF2)7CF3)n (n=1, 2) and the corresponding triarylphosphites", JOURNAL OF FLUORINE CHEMISTRY, ELSEVIER, NL, vol. 119, no. 2, 1 February 2003 (2003-02-01), pages 141-149, XP004401781, ISSN: 0022-1139, DOI: 10.1016/S0022-1139(02)00242-7

## Description

### TECHNICAL FIELD

The present invention relates to a fluorine atom-containing phenol compound and more specifically relates to a fluorine atom-containing phenol compound having excellent compatibility with a fluorine-containing polymer and a fluorine-containing solvent.

### BACKGROUND ART

Phenol compounds are widely used in industrial applications such as polymerization inhibitors of radical polymerizable monomers, photographic developers, reducing agents, deterioration inhibitors of polymer materials, storage improvers of radical polymerizable compositions, synthesis intermediates of pharmaceutical products and industrial chemicals, metal surface modifiers, physiologically active substances and surface modifiers. Of these, a hindered phenol compound having a substituent on carbon adjacent to the substitution position of a phenolic hydroxyl group on a benzene ring is particularly useful in polymerization inhibitors of radical polymerizable monomers, deterioration inhibitors of polymer materials, storage improvers of radical polymerizable compositions and the like (Non-Patent Literature 1).

In recent years, fluorochemical materials (fluorine atom-containing materials) having functions such as durability improvement, surface modification and corrosion protection are industrially widely used. For example, polymer materials achieve higher functionality such as improvement of polymer durability through antioxidation by using fluorochemical materials (Patent Literature 1).

### CITATION LIST

### PATENT LITERATURE

Patent Literature 1: JP 6-41018 A

### NON-PATENT LITERATURE

Non-Patent Literature 1: Zenjiro OSAWA, Photostabilizing Technology of Polymers, CMC Publishing Co., Ltd. (1986)

### SUMMARY OF INVENTION

### TECHNICAL PROBLEMS

Depending on the structure, however, phenol compounds bleed out from the materials because of their low affinity for fluorine-containing polymers (fluororesins) and fluorine-containing solvents, and hinder production of coating liquids because of their low solubility, and it has been sought to improve such problems.

In view of the situation as described above, an object of the present invention is to provide a novel phenol compound having excellent affinity for a fluorine-containing polymer and a fluorine-containing solvent.

### SOLUTION TO PROBLEMS

Under these circumstances, the inventors of the present invention have made an intensive study and as a result found a novel phenol compound having a specific fluorine-containing alkyl group in the molecule and thus completed the present invention.

More specifically, the foregoing object is achieved by the following means.

### (1) A compound represented by formula (1) to be described later.

### ADVANTAGEOUS EFFECTS OF INVENTION

The present invention can provide a novel phenol compound having excellent affinity for (compatibility with) a fluorine-containing polymer and a fluorine-containing solvent.

Compounds represented by formula (1) to be described later include a specific fluorine-containing alkyl group in the molecule, and hence have excellent affinity for fluorine-containing polymers and fluorine-containing solvents and are particularly useful as polymerization inhibitors of radical polymerizable monomers, photographic developers, reducing agents, deterioration inhibitors of polymer materials, storage improvers of radical polymerizable compositions, synthesis intermediates of pharmaceutical products and industrial chemicals, metal surface modifiers, physiologically active substances, surface modifiers and the like.

### DESCRIPTION OF EMBODIMENTS

Preferred embodiments of a fluorine atom-containing phenol compound according to the invention are described below.

The characteristic features of the invention compared to the prior art are first described in detail.

Reasons why a phenol compound having a specific fluorine-containing alkyl group in the molecule according to the invention has excellent affinity for (compatibility with) fluorine-containing polymers and fluorine-containing solvents are not clarified in detail but the following mechanism is estimated. More specifically, the phenol compound of the invention is characterized by including a fluorine-containing alkyl group at a moiety having a phenol structure via a linking group. On the other hand, the phenol structure is substituted with an OH group having high polarizability. Having the characteristics as described above, this compound is more likely to form, in a fluorine-containing polymer or a fluorine-containing solvent, a micelle-like structure having a fluorine-containing alkyl group located on the surface side to increase the surface fluorine atom density and it is therefore estimated that the affinity for the fluorine-containing polymer and the fluorine-containing solvent is improved. From the structure and the linking mode of the fluorine functional group, the compound of the invention is deemed to have an unexpected effect that in particular the effect as described above is easily exhibited.

In addition, the phenol compound of the invention includes an alkylene group represented by X between an ester group and a perfluoroalkyl group in formula (1) to be described later. By the inclusion of the alkylene group, dissociation of an ester bond is less likely to occur even under high-temperature and high-humidity conditions and the compound is also excellent in stability.

### (Compound Represented by Formula (1))

A compound (fluorine atom-containing phenol compound) represented by formula (1) is described below in detail.

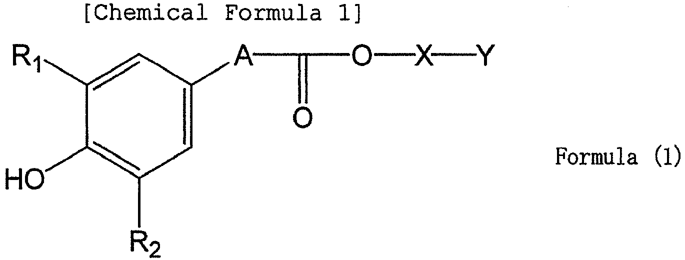

R₁ and R₂ each independently represent an alkyl group having 1 to 12 carbon atoms. The number of carbons in the alkyl group is preferably 1 to 8, more preferably 1 to 6 and most preferably 1 to 5 in terms of more excellent affinity for a fluorine-containing polymer and a fluorine-containing solvent. Specific examples of the suitable alkyl group include methyl, ethyl, n-propyl, isopropyl, t-butyl, isobutyl, 2,2-dimethylpropyl, hexyl and cyclohexyl.

A represents an alkylene group having 1 to 2 carbon atoms. A is preferably -CH₂- or -CH₂CH₂- and more preferably -CH₂CH₂-.

X represents an alkylene group having 1 to 3 carbon atoms which may contain a hydroxyl group. X is preferably -CH₂-,-CH₂CH₂-, -CH₂CH(CH₃)-, -CH₂CH₂CH₂-, -CH₂CH(OH)CH₂- or -CH₂CH(CH₂OH)-, more preferably -CH₂-, -CH₂CH₂-, -CH₂CH(OH)CH₂- or -CH₂CH₂CH₂-, and most preferably -CH₂- or -CH₂CH₂-.

Y represents a linear perfluoroalkyl group having 4 to 12 carbon atoms. Preferable examples of the perfluoroalkyl group include C₄F₉-, C₅F₁₁-, C₆F₁₃-, C₇F₁₅-, C₈F₁₇-, C₉F₁₉-, C₁₀F₂₁- and C₁₂F₂₅-.

The linear perfluoroalkyl group is inferior in affinity for a fluorine-containing polymer and a fluorine-containing solvent when the number of carbons is less than 4. Cases where the number of carbons exceeds 12 are not preferable in terms of economic efficiency because the affinity effect is saturated and a large number of fluorine atoms are contained.

R₁, R₂, A and X may each further have a substituent.

The substituent type is not particularly limited and the substituent represents, for example, any of the following: halogen atoms (e.g., chlorine atom, bromine atom and iodine atom); alkyl groups [(representing optionally substituted, linear, branched or cyclic alkyl groups including alkyl groups (preferably alkyl groups having 1 to 30 carbon atoms, such as methyl, ethyl, n-propyl, isopropyl, t-butyl, n-octyl, eicosyl, 2-chloroethyl, 2-cyanoethyl, and 2-ethylhexyl), cycloalkyl groups (preferably optionally substituted cycloalkyl groups having 3 to 30 carbon atoms, such as cyclohexyl, cyclopentyl, and 4-n-dodecylcyclohexyl), and bicycloalkyl groups (preferably optionally substituted bicycloalkyl groups having 5 to 30 carbon atoms, i.e., monovalent groups obtained by removing one hydrogen atom from bicycloalkanes having 5 to 30 carbon atoms, such as bicyclo[1,2,2]heptan-2-yl, bicyclo[2,2,2]octan-3-yl) and also including tricyclo structures each containing a large number of cyclic structures; alkyl groups in substituents to be illustrated below (e.g., an alkyl group in an alkylthio group) also representing the alkyl groups of the concept given above];

alkenyl groups [representing optionally substituted, linear, branched or cyclic alkenyl groups including alkenyl groups (preferably optionally substituted alkenyl groups having 2 to 30 carbon atoms, such as vinyl, allyl, prenyl, geranyl, and oleyl), cycloalkenyl groups (preferably optionally substituted cycloalkenyl groups having 3 to 30 carbon atoms, i.e., monovalent groups obtained by removing one hydrogen atom from cycloalkenes having 3 to 30 carbon atoms, such as 2-cyclopenten-1-yl, and 2-cyclohexen-1-yl), and bicycloalkenyl groups (optionally substituted bicycloalkenyl groups, preferably optionally substituted bicycloalkenyl groups having 5 to 30 carbon atoms, i.e., monovalent groups obtained by removing one hydrogen atom from bicycloalkenes having a double bond, including, for example, bicyclo[2,2,1]hept-2-en-1-yl, and bicyclo[2,2,2]oct-2-en-4-yl)], alkynyl groups (preferably optionally substituted alkynyl groups having 2 to 30 carbon atoms, such as ethynyl, propargyl and trimethylsilylethynyl groups);

aryl groups (preferably optionally substituted aryl groups having 6 to 30 carbon atoms, such as phenyl, p-tolyl, naphthyl, m-chlorophenyl, and o-hexadecanoylaminophenyl), heterocyclic groups (preferably monovalent groups obtained by removing one hydrogen atom from optionally substituted, 5- or 6-membered, aromatic or non-aromatic heterocyclic compounds, and more preferably 5- or 6-membered heteroaromatic ring groups having 3 to 30 carbon atoms, such as 2-furanyl, 2-thienyl, 2-pyrimidinyl and 2-benzothiazolyl);

cyano group, hydroxyl group, nitro group, carboxyl group, alkoxy groups (preferably optionally substituted alkoxy groups having 1 to 30 carbon atoms, such as methoxy, ethoxy, isopropoxy, t-butoxy, n-octyloxy, and 2-methoxyethoxy), aryloxy groups (preferably optionally substituted aryloxy groups having 6 to 30 carbon atoms, such as phenoxy, 2-methylphenoxy, 4-t-butylphenoxy, 3-nitrophenoxy, and 2-tetradecanoylaminophenoxy), silyloxy groups (preferably silyloxy groups having 3 to 20 carbon atoms, such as trimethylsilyloxy, and t-butyldimethylsilyloxy), heterocyclic oxy groups (preferably optionally substituted heterocyclic oxy groups having 2 to 30 carbon atoms, such as 1-phenyltetrazol-5-oxy, and 2-tetrahydropyranyloxy), acyloxy groups (preferably formyloxy group, optionally substituted alkylcarbonyloxy groups having 2 to 30 carbon atoms, and optionally substituted arylcarbonyloxy groups having 6 to 30 carbon atoms, such as formyloxy, acetyloxy, pivaloyloxy, stearoyloxy, benzoyloxy, and p-methoxyphenylcarbonyloxy), carbamoyloxy groups (preferably optionally substituted carbamoyloxy groups having 1 to 30 carbon atoms, such as N,N-dimethylcarbamoyloxy, N,N-diethylcarbamoyloxy, morpholinocarbonyloxy, N,N-di-n-octylaminocarbonyloxy, and N-n-octylcarbamoyloxy), alkoxycarbonyloxy groups (preferably optionally substituted alkoxycarbonyloxy groups having 2 to 30 carbon atoms, such as methoxycarbonyloxy, ethoxycarbonyloxy, t-butoxycarbonyloxy, and n-octylcarbonyloxy), aryloxycarbonyloxy groups (preferably optionally substituted aryloxycarbonyloxy groups having 7 to 30 carbon atoms, such as phenoxycarbonyloxy, p-methoxyphenoxycarbonyloxy, and p-n-hexadecyloxyphenoxycarbonyloxy);

amino groups (preferably amino group, optionally substituted alkylamino groups having 1 to 30 carbon atoms, and optionally substituted anilino groups having 6 to 30 carbon atoms, such as amino, methylamino, dimethylamino, anilino, N-methyl-anilino and diphenylamino), acylamino groups (preferably formylamino group, optionally substituted alkylcarbonylamino groups having 1 to 30 carbon atoms, and optionally substituted arylcarbonylamino groups having 6 to 30 carbon atoms, such as formylamino, acetylamino, pivaloylamino, lauroylamino, benzoylamino, and 3,4,5-tri-n-octyloxyphenylcarbonylamino), aminocarbonylamino groups (preferably optionally substituted aminocarbonylamino having 1 to 30 carbon atoms, such as carbamoylamino, N,N-dimethylaminocarbonylamino, N,N-diethylaminocarbonylamino, and morpholinocarbonylamino), alkoxycarbonylamino groups (preferably optionally substituted alkoxycarbonylamino groups having 2 to 30 carbon atoms, such as methoxycarbonylamino, ethoxycarbonylamino, t-butoxycarbonylamino, n-octadecyloxycarbonylamino, and N-methyl-methoxycarbonylamino), aryloxycarbonylamino groups (preferably optionally substituted aryloxycarbonylamino groups having 7 to 30 carbon atoms, such as phenoxycarbonylamino, p-chlorophenoxycarbonylamino, and m-n-octyloxyphenoxycarbonylamino), sulfamoylamino groups (preferably optionally substituted sulfamoylamino groups having 0 to 30 carbon atoms, such as sulfamoylamino, N,N-dimethylaminosulfonylamino, and N-n-octylaminosulfonylamino), alkyl- and arylsulfonylamino groups (preferably optionally substituted alkylsulfonylamino having 1 to 30 carbon atoms, and optionally substituted arylsulfonylamino having 6 to 30 carbon atoms, such as methylsulfonylamino, butylsulfonylamino, phenylsulfonylamino, 2,3,5-trichlorophenylsulfonylamino, and p-methylphenylsulfonylamino);

mercapto group, alkylthio groups (preferably optionally substituted alkylthio groups having 1 to 30 carbon atoms, such as methylthio, ethylthio, and n-hexadecylthio), arylthio groups (preferably optionally substituted arylthio having 6 to 30 carbon atoms, such as phenylthio, p-chlorophenylthio, and m-methoxyphenylthio), heterocyclic thio groups (preferably optionally substituted heterocyclic thio groups having 2 to 30 carbon atoms, such as 2-benzothiazolylthio, and 1-phenyltetrazol-5-ylthio), sulfamoyl groups (preferably optionally substituted sulfamoyl groups having 0 to 30 carbon atoms, such as N-ethylsulfamoyl, N-(3-dodecyloxypropyl)sulfamoyl, N,N-dimethylsulfamoyl, N-acetylsulfamoyl, N-benzoylsulfamoyl, and N-(N'-phenylcarbamoyl)sulfamoyl), sulfo group, alkyl- and arylsulfinyl groups (preferably optionally substituted alkylsulfinyl groups having 1 to 30 carbon atoms, and optionally substituted arylsulfinyl groups having 6 to 30 carbon atoms, such as methylsulfinyl, ethylsulfinyl, phenylsulfinyl and p-methylphenylsulfinyl);

alkyl- and arylsulfonyl groups (preferably optionally substituted alkylsulfonyl groups having 1 to 30 carbon atoms, and optionally substituted arylsulfonyl groups having 6 to 30 carbon atoms, such as methylsulfonyl, ethylsulfonyl, phenylsulfonyl, and p-methylphenylsulfonyl), acyl groups (preferably formyl group, optionally substituted alkylcarbonyl groups having 2 to 30 carbon atoms, optionally substituted arylcarbonyl groups having 7 to 30 carbon atoms, and optionally substituted heterocyclic carbonyl groups having 4 to 30 carbon atoms in which a carbonyl group is bonded to a carbon atom, such as acetyl, pivaloyl, 2-chloroacetyl, stearoyl, benzoyl, p-n-octyloxyphenylcarbonyl, 2-pyridylcarbonyl, and 2-furylcarbonyl), aryloxycarbonyl groups (preferably optionally substituted aryloxycarbonyl groups having 7 to 30 carbon atoms, such as phenoxycarbonyl, o-chlorophenoxycarbonyl, m-nitrophenoxycarbonyl, and p-t-butylphenoxycarbonyl), alkoxycarbonyl groups (preferably optionally substituted alkoxycarbonyl groups having 2 to 30 carbon atoms, such as methoxycarbonyl, ethoxycarbonyl, t-butoxycarbonyl, and n-octadecyloxycarbonyl); and

carbamoyl groups (preferably optionally substituted carbamoyl having 1 to 30 carbon atoms, such as carbamoyl, N-methylcarbamoyl, N,N-dimethylcarbamoyl, N,N-di-n-octylcarbamoyl, and N-(methylsulfonyl)carbamoyl), aryl- and heterocyclic azo groups (preferably optionally substituted arylazo groups having 6 to 30 carbon atoms, and optionally substituted heterocyclic azo groups having 3 to 30 carbon atoms, such as phenylazo, p-chlorophenylazo, and 5-ethylthio-1,3,4-thiadiazol-2-ylazo), imide groups (preferably N-succinimide, and N-phthalimide), phosphino groups (preferably optionally substituted phosphino groups having 2 to 30 carbon atoms, such as dimethylphosphino, diphenylphosphino, and methylphenoxyphosphino), phosphinyl groups (preferably optionally substituted phosphinyl groups having 2 to 30 carbon atoms, such as phosphinyl, dioctyloxyphosphinyl, and diethoxyphosphinyl), phosphinyloxy groups (preferably optionally substituted phosphinyloxy groups having 2 to 30 carbon atoms, such as diphenoxyphosphinyloxy, and dioctyloxyphosphinyloxy), phosphinylamino groups (preferably optionally substituted phosphinylamino groups having 2 to 30 carbon atoms, such as dimethoxyphosphinylamino, and dimethylaminophosphinylamino), silyl groups (preferably optionally substituted silyl groups having 3 to 30 carbon atoms, such as trimethylsilyl, t-butyldimethylsilyl, and phenyldimethylsilyl).

Of the foregoing functional groups, ones having a hydrogen atom may be further substituted with any of the foregoing groups after removal of the hydrogen atom. Examples of such functional groups include alkylcarbonylaminosulfonyl groups, arylcarbonylaminosulfonyl groups, alkylsulfonylaminocarbonyl groups, and arylsulfonylaminocarbonyl groups. Exemplary groups include methylsulfonylaminocarbonyl, p-methylphenylsulfonylaminocarbonyl, acetylaminosulfonyl, and benzoylaminosulfonyl groups.

Examples of the compound represented by formula (1) according to the invention are illustrated below but the present invention is not limited thereto.

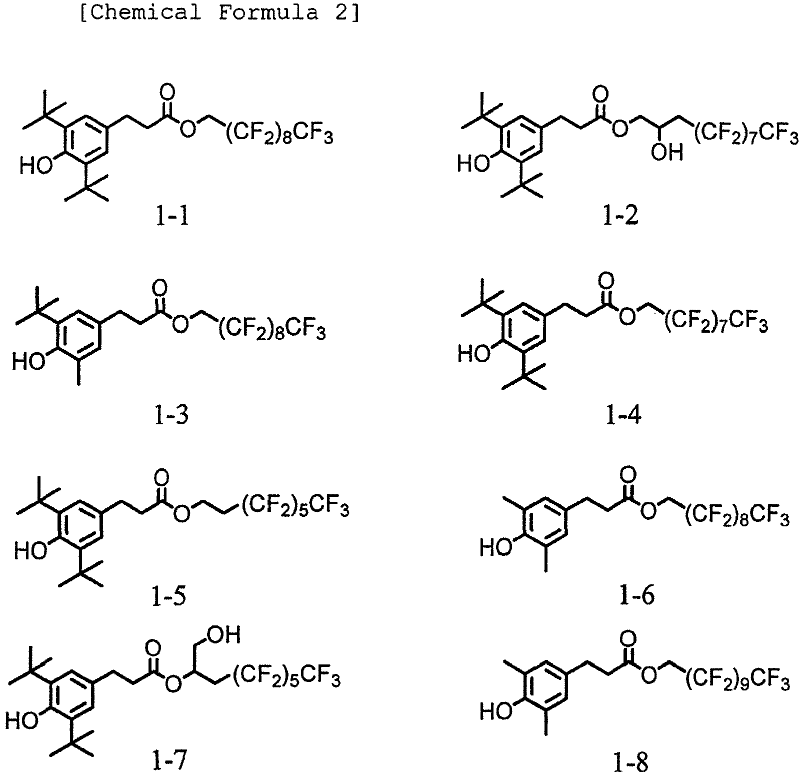

### (Method of Producing Compound Represented by Formula (1))

The method of producing the compound represented by formula (1) is not particularly limited and the compound represented by formula (1) can be produced by combining known methods.

For example, the compound represented by formula (1) can be produced by a step shown in Scheme 1 or Scheme 2 shown below but the production method is not limited thereto.

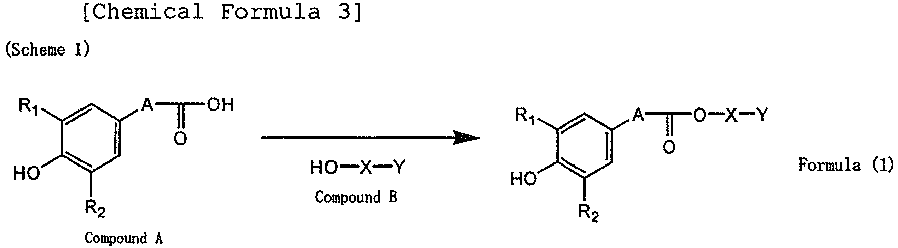

As shown in Scheme 1, Compound A having a carboxylic acid group and Compound B having a hydroxyl group are prepared and esterification is carried out, whereby a desired compound represented by formula (1) can be synthesized.

A method for esterification can be selected from known methods such as a method using an acid catalyst (e.g., sulfuric acid, methanesulfonic acid, or p-toluenesulfonic acid), and a method using a condensation agent (e.g., 1,3-dicyclohexylcarbodiimide, or 1-ethyl-3-(3-dimethylaminopropyl)carbodiimide hydrochloride).

It should be noted that the foregoing reaction may be carried out in the presence of a solvent if necessary. The type of the solvent to be used is not particularly limited and examples thereof include water and organic solvents.

Next, unreacted materials, by-products and other impurities are separated for refinement if necessary to obtain the compound represented by formula (1). Separation and refinement need only be performed by a common method, and examples thereof include an extraction operation using an organic solvent, recrystallization, crystallization using a poor solvent, and column chromatography using silica gel.

R₁, R₂ and A of Compound A, and X and Y of Compound B in Scheme 1 are as defined above.

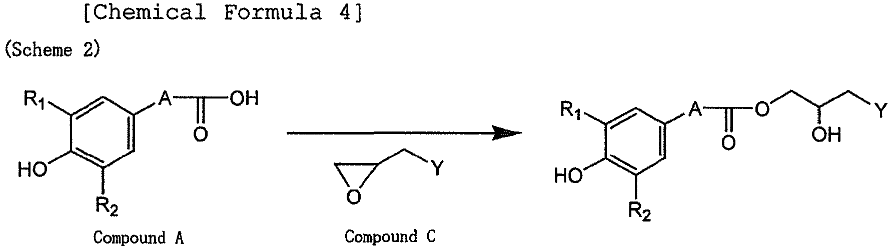

As shown in Scheme 2, Compound A having a carboxylic acid group and Compound C (epoxide) having an epoxy group are prepared and a ring-opening reaction of the epoxy group is carried out, whereby a desired compound corresponding to the compound represented by formula (1) can be synthesized.

The ring-opening reaction of the epoxy group is carried out in the presence of a known catalyst if necessary. Any known compound can be used as the catalyst and the catalyst is suitably selected from among, for example, organic bases (e.g., triethylamine, trimethylamine, diisopropylethylamine, pyridine, morpholine, 1,4-diazabicyclo[2.2.2]octane, 1,5-diazabicyclo[4.3.0]non-5-ene, 1,8-diazabicyclo[5.4.0]undec-7-ene, sodium methoxide, potassium methoxide, sodium ethoxide, and potassium ethoxide), quaternary ammonium salts (e.g., tetraethylammonium bromide, tetrabutylammonium bromide, tetrahexylbutylammonium bromide, and trimethyloctylammonium bromide), and triphenylphosphine.

It should be noted that the foregoing reaction may be carried out in the presence of a solvent if necessary. The type of the solvent to be used is not particularly limited and examples thereof include water and organic solvents.

Various types of separation and refinement described for Scheme 1 may be carried out after the end of the reaction if necessary.

R₁, R₂ and A of Compound A and Y of Compound C in Scheme 2 are as defined above.

The compound represented by formula (1) can be used in various applications.

In addition, the compound represented by formula (1) has excellent affinity for a fluorine-containing polymer (fluororesin) and a fluorine-containing solvent. Examples of the fluorine-containing polymer include known fluorine atom-containing polymers (e.g., polytetrafluoroethylene, polyvinylidene fluoride, polyvinyl fluoride, and a cyclized polymer of perfluoro(butenylvinylether) (Cytop (registered trademark))). The fluorine-containing polymer may also be a polymer obtained by polymerizing a fluorine-containing ethylenic monomer. Examples of the fluorine-containing ethylenic monomer include vinylidene fluoride, tetrafluoroethylene, hexafluoropropylene, vinyl trifluorochloride, vinyl fluoride, perfluoroalkyl vinyl ether, fluorine-containing (meth)acrylic monomers (e.g., 1H,1H,2H,2H-heptadecafluorodecyl methacrylate, 1H,1H,5H-octafluoropentyl methacrylate, 2,2,3,3-tetrafluoropropyl methacrylate, 2,2,2-trifluoroethyl methacrylate, 1H,1H,2H,2H-heptadecafluorodecyl acrylate, 1H,1H,5H-octafluoropentyl acrylate, 2,2,3,3-tetrafluoropropyl acrylate, 2,2,2-trifluoroethyl acrylate, perfluorooctylethyl methacrylate, and perfluorooctylethyl acrylate).

An example of the fluorine-containing solvent includes a known fluorine atom-containing solvent. Examples of the fluorine-containing solvent include a fluorine-modified aliphatic hydrocarbon solvent, a fluorine-modified aromatic hydrocarbon solvent, a fluorine-modified ether solvent, and a fluorine-modified alkylamine solvent. Specific examples of the fluorine-containing solvent that may be illustrated include polyfluorotrialkylamine compounds (fluorine-modified alkylamine solvents) such as perfluorobenzene, pentafluorobenzene, 1,3-bis(trifluoromethyl)benzene, 1,4-bis(trifluoromethyl)benzene, perfluorotributylamine, perfluorotripropylamine and perfluorotripentylamine; fluorine-modified aliphatic hydrocarbon solvents such as perfluorodecalin, perfluorocyclohexane, perfluoro(1,3,5-trimethylcyclohexane), perfluoro(2-butyltetrahydrofuran), perfluorohexane, perfluorooctane, perfluorodecane, perfluorododecane, perfluoro(2,7-dimethyloctane), 1,1,2-trichloro-1,2,2-trifluoroethane, 1,1,1-trichloro-2,2,2-trifluoroethane, 1,3-dichloro-1,1,2,2,3-pentafluoropropane, 1,1,1,3-tetrachloro-2,2,3,3-tetrafluoropropane, 1,1,3,4-tetrachloro-1,2,2,3,4,4-hexafluorobutane, perfluoro(1,2-dimethylhexane), perfluoro(1,3-dimethylhexane), 2H,3H-perfluoropentane, 1H-perfluorohexane, 1H-perfluorooctane, 1H-perfluorodecane, 1H,1H,1H,2H,2H-perfluorohexane, 1H,1H,1H,2H,2H-perfluorooctane, 1H,1H,1H,2H,2H-perfluorodecane, 3H,4H-perfluoro-2-methylpentane, 2H,3H-perfluoro-2-methylpentane, 1H-1,1-dichloroperfluoropropane, 1H-1,3-dichloroperfluoropropane, and perfluoroheptane; fluorine-modified aromatic hydrocarbon solvents such as m-xylene trifluoride, m-xylene hexafluoride and benzotrifluoride; and fluorine-modified ether solvents such as methyl perfluorobutyl ether, and perfluoro(2-butyltetrahydrofuran).

### EXAMPLES

The present invention is described below in further detail by way of examples. However, the invention should not be construed as being limited to the following examples. Unless otherwise specified, the ratio is expressed by percentage by weight.

### <Example 1: Synthesis of Compound 1-1>

Compound 1-1 was synthesized according to the following scheme.

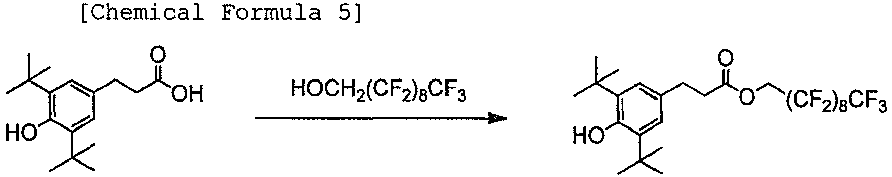

3-(3,5-Di-tert-butyl-4-hydroxyphenyl) propionic acid (3.5 g, 12.6 mmol), dichloromethane (20 mL), 2, 2, 3, 3, 4, 4, 5, 5, 6, 6, 7, 7, 8, 8, 9, 9, 10, 10, 10-nonadecafluorodecan-1-ol (6.3 g, 12.6 mmol), tetrahydrofuran (10 mL), 1-ethyl-3-(3-dimethylaminopropyl)carbodiimide hydrochloride (2,4 g, 12.6 mmol), and 4-dimethylaminopyridine (0.05 g, 0.4 mmol) were added to a reaction vessel in this order.

The reaction solution was stirred at room temperature for 3 hours and 1 N hydrochloric acid (50 mL) was then added to the reaction solution, which was followed by extraction with 100 mL of ethyl acetate. The organic layer was washed with a saturated saline solution and dried over magnesium sulfate. Solids were separated by filtration and the filtrate was concentrated under reduced pressure to obtain white crude crystals. The crystals were recrystallized in methanol to obtain 6.0 g of the foregoing Compound 1-1 (yield: 63%).

The resulting Compound 1-1 has the following ¹H-NMR spectrum:
¹H-NMR (solvent: deuterated chloroform; reference: tetramethylsilane)
   6.98(2H,s), 5.09(1H,s), 4.59(2H,t), 2.90(2H,t), 2.71(2H,t), 1.43(9H,s)

The resulting compound was identified as Compound 1-1 since each proton peak was observed at a characteristic position in the ¹H-NMR data.

### <Example 2: Synthesis of Compound 1-2>

Compound 1-2 was synthesized according to the following scheme.

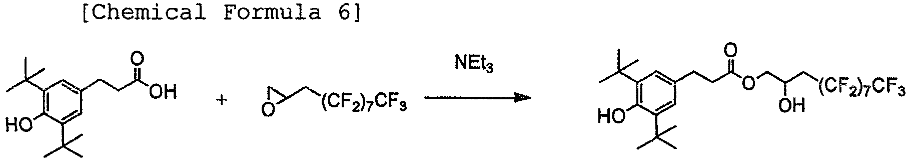

3-(3,5-Di-tert-butyl-4-hydroxyphenyl) propionic acid (3.3 g, 12 mmol), and 2-(2,2,3,3,4,4,5,5,6,6,7,7,8,8,9,9,9-heptadecafluorononyl)oxirane (4.8 g, 10 mmol) were put in a reaction vessel and dissolved by addition of methyl isobutyl ketone (5 g). Triethylamine (100 mg, 1 mmol) was further added to the reaction solution and the reaction solution was reacted at 100°C for 6 hours. Then, the reaction mixture was cooled to room temperature and the solution was concentrated under reduced pressure. The solution was purified by silica gel column chromatography (mobile phase: hexane / ethyl acetate = 4/1) to obtain 5.5 g of the foregoing Compound 1-2 (yield: 74%).

The resulting Compound 1-2 has the following NMR spectrum:
¹H-NMR (solvent: deuterated chloroform; reference: tetramethylsilane)
   6.98(2H,s), 5.09(1H,s), 4.31(1H,m) 4.23(1H,dd), 4.07(1H,dd), 2.90(2H,t), 2.69(2H,t), 2.30(2H,m), 1.40(9H,s)

The resulting compound was identified as Compound 1-2 since each proton peak was observed at a characteristic position in the ¹H-NMR data.

Compounds 1-3 to 1-8 illustrated above as specific examples of the compound represented by formula (1) were synthesized according to the same procedure as in Example 1 or 2.

### <Solubility Check>

### (Testing Method)

To a mixed solution of perfluorotributylamine / 1,1,1,3,3,3-hexafluoropropan-2-ol / Cytop CTL-809M (manufactured by Asahi Glass Co., Ltd.) = 91/5/4 (parts by weight), was added the compound represented by formula (1) according to the invention (any of Compounds 1-1 to 1-8) or Comparative Compound C-1 or C-2 as shown below in an amount of 0.3 wt% with respect to the Cytop CTL-809M. Thereafter, the mixed solution was applied onto a glass substrate to a film thickness of 1.5 µm and dried.

The surface profile of the resulting coated film was observed by an optical microscope to check whether each compound bled out or remained partially undissolved. The results are compiled in Table 1.

Compounds which neither bleed out nor remain partially undissolved are denoted by "Compatible," a compound which bleeds out is denoted by "Bleed out," and a compound which remains partially undissolved is denoted by "Remain partially undissolved."

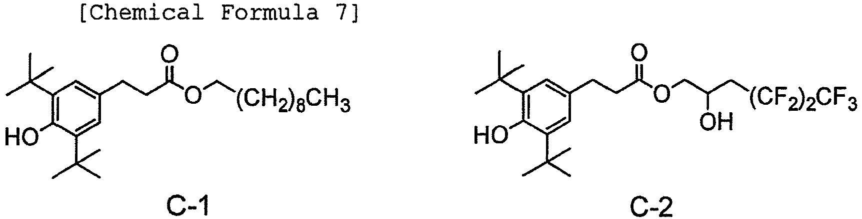

**[Table 1]**

| Table 1 | | |
|---|---|---|
| Compound | Solubility | Remark |
| Compound 1-1 | Compatible | Present Invention |
| Compound 1-2 | Compatible | Present Invention |
| Compound 1-3 | Compatible | Present Invention |
| Compound 1-4 | Compatible | Present Invention |
| Compound 1-5 | Compatible | Present Invention |
| Compound 1-6 | Compatible | Present Invention |
| Compound 1-7 | Compatible | Present Invention |
| Compound 1-8 | Compatible | Present Invention |
| Compound C-1 | Remain partially undissolved | Comparative Example |
| Compound C-2 | Bleed out | Comparative Example |

When Comparative Compound C-1 free from a fluorine-containing alkyl group was used, the compound remained partially undissolved. When Comparative Compound C-2 which included a fluorine-containing alkyl group but fell outside the scope of this application was used, the compound bled out on the resin surface.

In contrast, the compounds of the invention are found to exhibit excellent compatibility with fluororesin and to have high affinity for fluorine materials. From the above, the beneficial effects of the invention are obvious.

## Claims

1. A compound represented by formula (1) shown below: 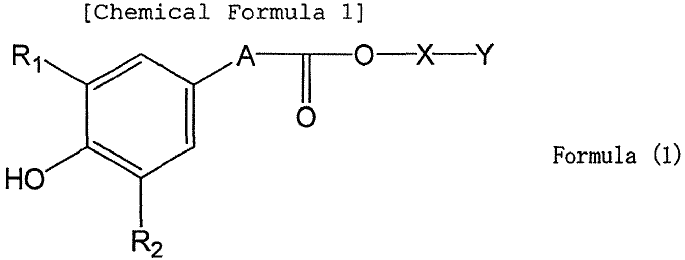 (in formula (1), R₁ and R₂ each independently represent an alkyl group having 1 to 12 carbon atoms; A represents an alkylene group having 1 to 2 carbon atoms; X represents an alkylene group having 1 to 3 carbon atoms optionally containing a hydroxyl group; and Y represents a linear perfluoroalkyl group having 4 to 12 carbon atoms).

## Patentansprüche

1. Verbindung, dargestellt durch die nachstehend dargestellte Formel (1): 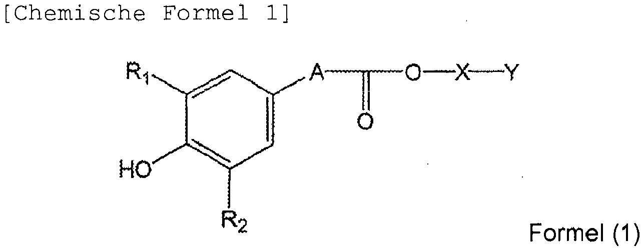 (in Formel (1) stellen R₁ und R₂ unabhängig eine Alkylgruppe mit 1 bis 12 Kohlenstoffatomen dar; A stellt eine Alkylengruppe mit 1 bis 2 Kohlenstoffatomen dar; X stellt eine Alkylengruppe mit 1 bis 3 Kohlenstoffatomen dar, die gegebenenfalls eine Hydroxylgruppe enthält; und Y stellt eine geradkettige Perfluoralkylgruppe mit 4 bis 12 Kohlenstoffatomen dar).

## Revendications

1. Composé représenté par la formule (1) indiquée ci-dessous : 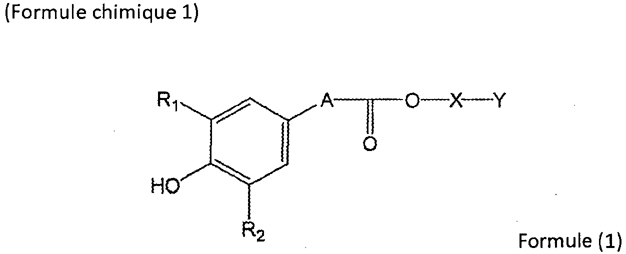 (dans la formule (1), R₁ et R₂ représentent chacun indépendamment un groupe alkyle ayant 1 à 12 atomes de carbone ; A représente un groupe alkylène ayant 1 à 2 atomes de carbone ; X représente un groupe alkylène ayant 1 à 3 atomes de carbone contenant éventuellement un groupe hydroxyle ; et Y représente un groupe perfluoroalkyle linéaire ayant 4 à 12 atomes de carbone).
